# EUROPÄISCHE PATENTSCHRIFT

(11) **EP 0 648 739 B1**
(45) Veröffentlichungstag und Bekanntmachung des Hinweises auf die Patenterteilung: **12.03.1997**
(21) Anmeldenummer: 94116057.4
(22) Anmeldetag: 12.10.1994
(51) Int. Cl.: C07C 239/20, C08F 220/10, C08F 246/00, C07C 271/10, C07C 271/18

(54) **Oximether und diese enthaltende Copolymerisate**
Oximether and copolymerisates containing them
Oximéther et copolymérisats les contenant

(30) Priorität: 19.10.1993 DE 4335555
(43) Veröffentlichungstag der Anmeldung: 19.04.1995
(73) Patentinhaber: BASF Aktiengesellschaft, 67063 Ludwigshafen (DE)
(72) Erfinder: Bauer, Gerhard, Dr., D-69469 Weinheim (DE); Bott, Kaspar, Dr., D-68165 Mannheim (DE)

(56) Entgegenhaltungen:
- EP-A- 0 516 074
- EP-A- 0 617 013
- WO-A-93/25519
- WO-A-93/25588
- CH-A- 419 169

## Beschreibung

Die Erfindung betrifft Hydroxylaminderivate der allgemeinen Formel 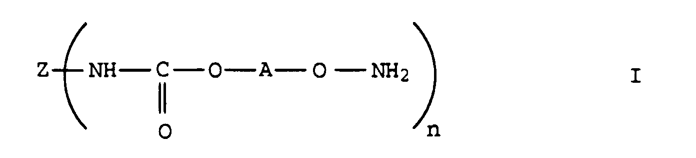 oder 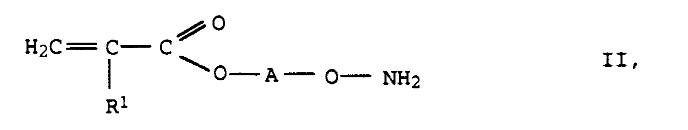 worin A für ein zweiwertiges Bindeglied steht, R¹ ein Wasserstoffatom oder eine C₁-C₄-Alkylgruppe sein kann, Z für einen n-wertigen organischen Rest steht, der eine copolymerisierbare ethylenisch ungesättigte Gruppe enthält und n eine ganze Zahl von 1 bis 3 ist, und deren Salze.

Weiterhin betrifft die Erfindung ein Verfahren zur Herstellung der Hydroxylaminderivate und deren Salze sowie Copolymerisate, welche die Hydroxylaminderivate enthalten.

Bei Copolymerisaten, welche in Beschichtungsmitteln oder Klebstoffen Verwendung finden, handelt es sich vielfach um vernetzungsfähige Copolymerisate. Durch eine Vernetzung können z.B. Schutzüberzüge oder Klebstoffbeschichtungen mit guten elastischen Eigenschaften, hoher Kohäsion, hoher Chemikalien- und Lösemittelbeständigkeit erhalten werden.

Zur Vernetzung wird den Copolymerisaten im allgemeinen ein Vernetzungsmittel zugesetzt, das mit funktionellen Gruppen im Copolymerisat reagiert. Mögliche Vernetzungsmittel sind z.B. Polyisocyanate, welche mit Hydroxylgruppen oder Aminogruppen reagieren.

Aus der DE-A-35 21 618 sind entsprechende wäßrige Klebstoffzubereitungen bekannt, bei denen in Wasser dispergierte Polyisocyanate wäßrigen Dispersionen radikalisch polymerisierter Copolymerisate als Vernetzungsmittel zugesetzt werden. Ähnliche Klebstoffzubereitungen sind auch in der US-A-43 96 738 und DE-A-31 12 117 beschrieben.

Nachteilig bei diesen wäßrigen Zubereitungen ist jedoch die mangelnde Lagerstabilität. Das Polyisocyanat darf daher erst kurz vor seiner Verwendung als Vernetzungshilfsmittel in Wasser dispergiert und mit dem Copolymerisat gemischt werden.

Eine erhöhte Lagerstabilität kann durch Umsetzung der Isocyanatgruppen mit Blockierungsmitteln, z.B. Oximen, Caprolactam, Phenolen, Maleinsäuredialkylestern erreicht werden. Die erhaltenen sog. blockierten Polyisocyante hydrolysieren in wäßriger Dispersion nur noch in untergeordnetem Ausmaß.

Gegenstand der DE-A-38 07 555 ist ein solches, mit Oximen blockiertes Diisocyanat, welches in Wasser dispergiert wird und sich als Zusatz für in Wasser dispergierte Polymerisate eignet.

Vernetzungsreaktionen treten bei den Systemen dieser Schrift jedoch erst nach Abspaltung des Blockierungsmittels bei Temperaturen ab ca. 130°C auf.

Bisher bekannte wäßrige Klebstoffzubereitungen mit Polyisocyanaten als Vernetzungsmittel sind daher entweder nicht lagerstabil und können daher nur als 2-Komponentensystem Verwendung finden oder vernetzen erst bei hohen Temperaturen.

Lagerstabile, bei Raumtemperatur nach Entfernen des Lösungsmittels vernetzende wäßrige Dispersionen sind aus der EP-A-3516 bekannt. Diese Dispersionen enthalten Polyhydrazide, welche mit im Copolymerisat einpolymerisieren Monomeren mit Carbonylgruppen reagieren.

Des weiteren sind aus der EP-A-516 074 Dispersionen bekannt, welche Aminoxyvernetzer enthalten. In den deutschen Patentanmeldungen P 41 21 946.5 (≙ EP-A-522 306) bzw. P 42 19 385.0 und P 43 09 193.8 sind mit Oxim blockierte Polyisocyanate bzw. copolymerisierbare Oximether als Vernetzungsmittel bekannt. Eine Vernetzung tritt jeweils ein mit Carbonylgruppen enthaltenden Copolymerisaten. In der deutschen Patentanmeldung P 43 14 623.6 sind nicht copolymerisierbare Hydroxylamine beschrieben.

Es ist grundsätzlich erwünscht, weitere lagerstabile, bei Raumtemperatur vernetzende Dispersionen zu entwickeln, um Alternativen zur Polyhydrazidvernetzung zur Verfügung zu stellen. Insbesondere sind auch vernetzende Monomere gewünscht, die copolymerisiert werden können und so nicht z.B. aus Beschichtungsmassen entweichen können.

Aufgabe der vorliegenden Erfindung waren daher vernetzbare Copolymerisate, welche in Dispersion oder Lösung auch in Gegenwart eines Vernetzungsmittels lagerstabil sind und bei Raumtemperatur vernetzt werden können.

Demgemäß wurden die oben definierten Hydroxylaminderivate und deren Salze und ein Verfahren zu ihrer Herstellung gefunden.

Des weiteren wurden Copolymerisate, welche die genannten Hydroxylaminderivate in freier Form oder in Salzform einpolymerisiert enthalten und die Verwendung der Copolymerisate als Beschichtungsmittel oder Klebstoff gefunden.

Die Copolymerisate, welche die Hydroxylaminderivate enthalten, zeigen eine gute Haftung, auch im noch nassen Zustand (Naßhaftung), auf unterschiedlichsten Substraten und vernetzen insbesondere mit Aldehyd- oder Ketogruppen enthaltenden Verbindungen.

Bei der Variablen A in der allgemeinen Formel I und II handelt es sich bevorzugt um eine lineare oder verzweigte Kohlenwasserstoffkette aus 2 bis 12, insbesondere 2 bis 8 Kohlenstoffatomen, welche gegebenenfalls durch 1 bis 3, insbesondere 1 - 2 nicht benachbarte Schwefel- oder Stickstoffatome, vorzugsweise Sauerstoffatome oder einen C₅-C₁₀-Cycloalkylen- oder einen C₅-C₁₀-Arylenring unterbrochen sein kann. Besonders bevorzugt handelt es sich um eine lineare oder verzweigte Kohlenwasserstoffkette aus 2 bis 8 Kohlenstoffatomen.

R¹ steht bevorzugt für ein Wasserstoffatom oder eine Methylgruppe, n steht für eine ganze Zahl von 1 bis 3, vorzugsweise steht n für 1.

Z steht für einen organischen Rest, der eine copolymerisierbare ethylenisch ungesättigte Gruppe enthält.

Bei Z kann es sich um als solche bekannte Monomere für die radikalische Polymerisation handeln, welche gemäß Formel I durch eine Gruppe 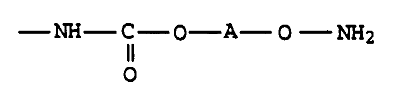 substituiert sind.

Als Z in Frage kommen z.B. vinylaromatische Reste mit bis zu 20 C-Atomen, (Meth)acrylsäureesterreste der Formel 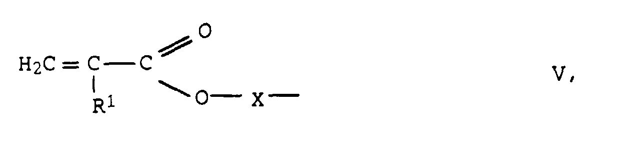 in der R¹ die obengenannte Bedeutung hat und X für ein organisches Bindeglied mit üblicherweise 1 bis 20 C-Atomen steht. Bevorzugt steht X für eine C₁-C₁₀-Alkylengruppe wie -CH₂-CH₂-.

Als Z in Betracht kommt daneben auch ein ethylenisch ungesättigter ein- oder mehrwertiger Rest mit einer Urethangruppe, der aus einem Mono- oder Polyisocyanat durch Abstraktion einer oder mehrerer Isocyanatgruppen entsteht. Die Urethangruppe im Rest Z ist üblicherweise durch Umsetzung eines Monomeren enthaltend eine isocyanatreaktive OH-Gruppe mit einer weiteren Isocyanatgruppe von Z erhältlich.

Besonders bevorzugt handelt es sich bei Z um eine Acroylgruppe oder Methacroylgruppe der Formel

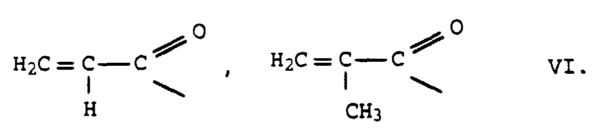

Die Hydroxylaminderivate der Formel I und II bzw. deren Salze sind erhältlich ausgehend von Oximethern der Formeln 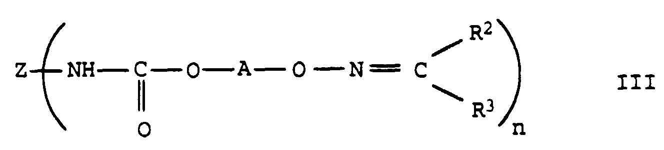 bzw. 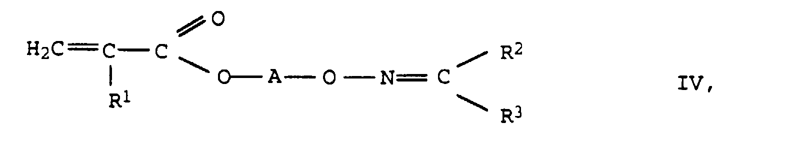 worin A, Z, n und R¹ die obengenannte Bedeutung haben und R² und R³ unabhängig voneinander für einen C₁- bis C₁₀-Alkyl-, einen C₁-C₁₀-Alkoxy, einen C₃- bis C₁₀-Cycloalkyl- oder einen C₅- bis C₁₀-Arylrest, welche auch 1 - 3 nicht benachbarte Stickstoff-, Sauerstoff- oder Schwefelatome als Heteroatome in der Kohlenstoffkette oder im Kohlenstoffring enthalten können und durch ein bis drei C₁- bis C₄-Alkyl- oder -Alkoxygruppen substituiert sein können, stehen, R² oder R³ für ein Wasserstoffatom stehen können oder R² und R³ gemeinsam eine Brücke aus 3 bis 14 Kohlenstoffatomen bilden, wobei ein Teil der Kohlenstoffatome auch Bestandteil eines aromatischen Rings sein kann.

R² und R³ stehen bevorzugt unabhängig voneinander für ein Wasserstoffatom, eine C₁- bis C₆-Alkylgruppe, C₁- bis C₆-Alkoxygruppe oder einen C₅-C₁₀-Arylrest, insbesondere einen Phenylring. Im Falle des Wasserstoffatoms kann nur einer der beiden Reste R² und R³ ein Wasserstoffatom sein. n steht für eine ganze Zahl von 1 bis 3, vorzugsweise steht n für 1. Besonders bevorzugt ist R² eine C₁- bis C₆-, insbesondere C₁- bis C₂-Alkylgruppe und R³ eine C₁- bis C₆-, insbesondere C₁- bis C₂-Alkoxygruppe.

Die Oximether der Formel III bzw. IV werden in Gegenwart von Wasser mit einer starken Säure mit einer Säurekonstanten >10⁻², bevorzugt >10⁻¹ (s. Anorganikum, Berlin, 1977, S. 458) zu den Hydroxylaminderivaten umgesetzt.

Die Hydroxylaminderivate I und II fallen hierbei in Form ihrer Salze an.

Zur Durchführung dieser Umsetzung läßt man die Oximether mit einer Mischung aus im allgemeinen Wasser und der entsprechenden Säure üblicherweise bei 0 bis 50°C, bevorzugt bei 20 bis 30°C reagieren. Dabei kommen jeweils 1 bis 4, vorzugsweise 1,5 bis 2 Äquivalente Säure pro Mol Oximether III bzw. IV zum Einsatz. Genannt seien Perchlorsäure, Schwefelsäure, aromatische oder aliphatische Sulfonsäuren, Phosphorsäure, Trifluoressigsäure und Halogenwasserstoffsäuren wie HCl oder HBr. Bevorzugt ist HCl.

Gute Ergebnisse wurden erzielt, wenn die Reaktion in einem Lösungsmittel wie Dioxan, Methanol, Ethanol, n-Propanol, Isopropanol oder Gemischen daraus durchgeführt wird und die entstandenen Salze durch ein Fällungsmittel wie Diethylether, Chlorethan oder Toluol ausgefällt werden.

Die freien Verbindungen der Formeln I und II können in bekannter Weise, soweit gewünscht, aus den Salzen durch geeignete Maßnahmen zur Deprotonierung wie Zugabe von Basen wie K₂CO₃, Na₂CO₃, NaOH oder aliphatische Amine erhalten werden. Salze oder Addukte anderer als der bisher genannten Säuren lassen sich wie üblich durch Umsetzung der freien Verbindungen mit den entsprechenden Säuren erzeugen. Genannt seien Kohlensäure, Essigsäure, Propionsäure oder Benzoesäure und insbesondere Ameisensäure.

Die Oximetherverbindungen der Formel III können hergestellt werden, wie in der deutschen Patentanmeldung P 43 09 193.8 beschrieben ist.

Die Herstellung der copolymerisierbaren Oximether der Formel III kann durch Umsetzung von Oximetheralkoholen der Formel 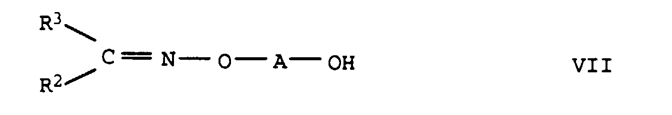 mit einer Isocyanatverbindung der Formel

Z(- N = C = O)ₙ VIII

erfolgen.

Die Umsetzung kann in einfacher Weise bei bevorzugt 0 bis 50, insbesondere 0 bis 20°C durch Zusammengeben der Ausgangsverbindungen, vorzugsweise im stöchiometrischen Verhältnis der Oximetheralkohole zu den Isocyanatgruppen erfolgen. Bevorzugt wird die Umsetzung in Gegenwart eines Lösungsmittels vorgenommen. Als Lösungsmittel zu nennen sind z.B. aromatische oder aliphatische Kohlenwasserstoffe, sowie Chlorkohlenwasserstoffe.

Die Oximetheralkohole der Formel VII als Ausgangsverbindungen für die Umsetzung sind nach bekannten Verfahren, z.B. durch Umsetzung von Oximen mit Alkylenoxiden wie Ethylenoxid, Propylenoxid etc. oder mit Halogenalkoholen in Gegenwart einer Base erhältlich.

Bei den Isocyanatverbindungen VIII handelt es sich üblicherweise um radikalisch polymerisierbare Monomere, also Verbindungen mit einer copolymerisierbaren ethylenisch ungesättigten Gruppe, welche mindestens eine Isocyanatgruppe enthalten.

Als Isocyanatverbindungen in Betracht kommen z.B. C₁-C₁₀-Alkyl-(meth)acrylate, die im Alkylrest durch mindestens eine, vorzugsweise eine Isocyanatgruppe substituiert sind, z.B. 2-Isocyanatoethyl(meth)acrylat und insbesondere Acryloylisocyanat und Methacryloylisocyanat.

Weitere Isocyanatverbindungen VIII können in einfacher Weise hergestellt werden, indem man zunächst Polyisocyanate, insbesondere Diisocyanate, mit ethylenisch ungesättigten Verbindungen umsetzt, so daß mindestens eine freie Isocyanatgruppe verbleibt. Als ethylenisch ungesättigte Verbindungen eignen sich solche, die mindestens eine gegenüber Isocyanat reaktive Gruppe, z.B. eine primäre oder sekundäre Aminogruppe oder vorzugsweise eine Hydroxylgruppe, aufweisen, beispielsweise 2-Hydroxyethyl(meth)acrylat oder p-Aminostyrol. Diese können in bekannter Weise mit einem Polyisocyanat unter Harnstoff- bzw. Urethanbildung umgesetzt werden. Vorzugsweise werden ethylenisch ungesättigte Verbindungen mit einer Hydroxygruppe, z.B. Hydroxy-C₂-C₁₀-alkyl(meth)acrylate, mit Polyisocyanaten, insbesondere Diisocyanaten, umgesetzt. Geeignete Diisocyanate sind z.B. solche mit der allgemeinen Formel X(NCO)₂, wobei X für einen aliphatischen Kohlenwasserstoffrest mit 4 bis 12 C-Atomen, einen cycloaliphatischen mit 6 bis 15 Atomen oder einen aromatischen oder alkaromatischen Kohlenwasserstoffrest mit 6 bis 15 C-Atomen steht. Genannt seien z.B. 1,4-Butandiisocyanat, 1,6-Hexandiisocyanat, 2,2,4- und 2,4,4-Trimethylhexamethylendiisocyanat, Cyclohexandiisocyanat, Methylcyclohexandiisocyanat, Isophorondiisocyanat, 4,4'-Diisocyanatodiphenylmethan, 4,4'-Diisocyanatodicyclohexylmethan, 2,4- und 2,6-Toluoldiisocyanat.

Die Oximetherverbindungen der Formel IV können hergestellt werden, wie in der deutschen Patentanmeldung P 42 19 385.0 beschrieben.

Sie können hergestellt werden z.B. durch Umesterung von Oximetheralkoholen der Formel VII mit Alkyl(meth)acrylaten, insbesondere den Methyl- oder Ethyl(meth)acrylaten.

Vorteilhaft ist jedoch die Umsetzung von Oximetheralkoholen mit (Meth)acrylsäurechloriden oder (Meth)acrylsäureanhydriden der Formeln 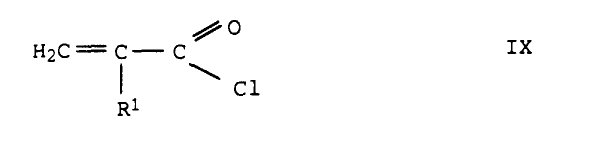 oder 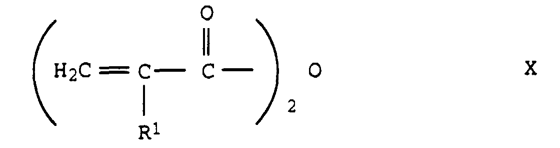 da die Reaktionszeiten kurz und die Reaktionstemperaturen niedrig gehalten werden können und so unerwünschte Nebenreaktionen wie Polymerisation und Zersetzung weitgehend vermieden werden.

Die Umsetzung der Oximetheralkohole VII mit (Meth-)acrylsäurechloriden IX erfolgt bevorzugt unter Kühlung der Reaktionsmischung bei Temperaturen zwischen 0 und 50°C, insbesondere 10 bis 30°C. Im Falle der (Meth)acrylsäureanhydride X sollte die Temperatur oberhalb von 60°C liegen. Die Umsetzung kann in einem organischen Lösungsmittel oder auch lösungsmittelfrei erfolgen. Als Lösungsmittel in Betracht zu ziehen sind z.B. Cyclohexan, Methylenchlorid, Diethylether, Methyl-tert-butylether, Ethylenchlorid.

Insbesondere sind auch in Hinsicht auf die weitere Aufarbeitung Ether, z.B. Diethylether als Lösungsmittel geeignet. Die Umsetzung wird bevorzugt in Gegenwart von Basen bzw. basisch reagierenden Verbindungen durchgeführt, die dabei als Säurefänger dienen. Als Basen bzw. basisch reagierende Verbindungen können z.B. tertiäre Stickstoffverbindungen wie Triethylamin eingesetzt werden. Die Base oder basisch reagierende Verbindung wird vorzugsweise in äquimolaren Mengen bezogen auf den Oximetheralkohol eingesetzt.

Nach Beendigung der Umsetzung kann die Reaktionsmischung z.B. mit einer wäßrigen Natriumcarbonatlösung gewaschen und der erhaltene Oximether gegebenenfalls nach Abtrennung des Lösungsmittels durch Destillation gereinigt werden.

Die Hydroxylaminderivate bzw. ihre Salze (im folgenden auch als Monomere a) bezeichnet) können nach üblichen Verfahren der radikalischen Polymerisation mit ethylenisch ungesättigten Monomeren copolymerisiert werden.

Für eine ausreichende Vernetzungsfähigkeit und gute Haftung der erhaltenen Copolymerisate sollte ihr Gehalt an einpolymerisierten Monomeren a) mindestens 0,01 Gew.-% betragen. Ein Gehalt über 30 Gew.-% ist im allgemeinen nicht notwendig.

Vorzugsweise ist der Gehalt an einpolymerisierten Monomeren a) im Copolymerisat 0,1 bis 10, besonders bevorzugt 0,1 bis 5 Gew.-%.

Der Anteil an Hauptmonomeren b) im Copolymerisat ist allgemeinen 30 - 99,99, vorzugsweise 70 bis 99,9, besonders bevorzugt 85 bis 99,9 Gew.-%. Das Hauptmonomere b) wird im allgemeinen ausgewählt aus C₁-C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigte Nitrile, Vinylhalogenide und nichtaromatischen Kohlenwasserstoffen mit mindestens 2 konjugierten Doppelbindungen.

Als Hauptmonomere zu nennen sind z.B. (Meth)acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, -stearat, Vinylpropionat und Vinylacetat.

Als vinylaromatische Verbindungen kommen Vinyltoluol, α- und p-Methylstyrol, α-Butylstyrol, 4-n-Butylstyrol, 4-n-Decylstyrol und vorzugsweise Styrol in Betracht. Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als nicht aromatische Kohlenwasserstoffe mit 2 bis 8 C-Atomen und mindestens zwei olefinischen Doppelbindungen seien Butadien, Isopren, und Chloropren genannt.

Die Hauptmonomeren werden auch vorzugsweise im Gemisch eingesetzt.

Die Copolymerisate können weiterhin auch Monomere mit mindestens einer Aldehyd- oder Ketogruppe (Monomere c)) enthalten.

Vorzugsweise handelt es sich um Monomere mit ein oder zwei Aldehyd-, bzw. Ketogruppen oder einer Aldehyd- und einer Ketogruppe und einer radikalisch polymerisierbaren olefinischen Doppelbindung.

In Betracht kommen z.B. Acrolein, Methacrolein, Vinylalkylketone mit 1 bis 20, vorzugsweise 1 bis 10 Kohlenstoffatomen im Alkylrest, Formylstyrol, (Meth-)acrylsäurealkylester mit ein oder zwei Keto- oder Aldehyd-, bzw. einer Aldehyd- und einer Ketogruppe im Alkylrest, wobei der Alkylrest vorzugsweise insgesamt 3 bis 10 Kohlenstoffatome umfaßt, z.B. (Meth)acroyloxyalkylpropanale, wie sie in der DE-A-27 22 097 beschrieben sind. Des weiteren eignen sich auch N-Oxoalkyl(meth)acrylamide wie sie z.B. aus der US-A-4 226 007, der DE-A-20 61 213 oder DE-A-22 07 209 bekannt sind.

Besonders bevorzugt sind Acetoacetyl(meth)acrylat, Acetoacetoxyethyl(meth)acrylat und insbesondere Diacetonacrylamid.

Der Gehalt dieser Monomeren beträgt im allgemeinen zwischen 0 und 30 Gew.-%, insbesondere zwischen 0 und 10, besonders bevorzugt zwischen 0 und 5 Gew.-%.

Das Copolymerisat kann selbst- oder fremdvernetzbar sein. Im Falle der Selbstvernetzbarkeit enthält es sowohl copolymerisierbare Oximether als auch vorzugsweise Monomere mit mindestens einer Keto- oder Aldehydgruppe. Die Vernetzung des Copolymerisats tritt dann ohne Zusatz eines Vernetzungsmittels durch Reaktion der Oximgruppe mit den Keto- oder Aldehydgruppe im gleichen Copolymerisat ein.

Der Gehalt am Monomer mit mindestens einer Keto- oder Aldehydgruppe c) im Copolymerisat sollte dann vorzugsweise mindestens 0,1 Gew.-% betragen. Die maximal mögliche Menge des Hauptmonomeren reduziert sich dann um 0,1 Gew.-%. Für eine gute Haftung ist ein Gehalt an Monomeren c) nicht notwendig.

Als weitere von den Monomeren a) bis c) verschiedene Monomere d), welche im Copolymerisat enthalten sein können, sind z.B. Ester der Acryl- und Methacrylsäure von Alkoholen mit 1 bis 20 C-Atomen, die außer dem Sauerstoffatom in der Alkoholgruppe mindestens ein weiteres Heteroatom enthalten und/oder die einen aliphatischen oder aromatischen Ring enthalten, wie 2-Ethoxyethylacrylat, 2-Butoxyethyl(meth)acrylat, Dimethylaminoethyl(meth)acrylat, Diethylaminoethyl(meth)acrylat, (Meth)acrylsäurearyl, -alkaryl- oder Cycloalkylester, wie Cyclohexyl(meth)acrylat, Phenylethyl-(meth)acrylat, Phenylpropyl(meth)acrylat oder Acrylsäureester von heterocyclischen Alkoholen wie Furfuryl(meth)acrylat genannt.

Darüber hinaus kommen noch weitere Monomere wie (Meth)acrylamid, sowie deren am Stickstoff mit C₁-C₄-Alkyl substituierten Derivate, in Betracht.

Von Bedeutung sind auch hydroxyfunktionelle Monomere, z.B. (Meth)acrylsäure-C₁-C₁₅-alkylester, welche durch ein oder zwei Hydroxygruppen substituiert sind. Insbesondere von Bedeutung als hydroxyfunktionelle Comonomere sind (Meth)acrylsäure-C₂-C₈-hydroxyalkylester, wie n-Hydroxyethyl-, n-Hydroxypropyl- oder n-Hydroxybutyl(meth)acrylat.

Die Mitverwendung von Comonomeren mit salzbildenden Gruppen empfiehlt sich insbesondere für die Herstellung selbstdispergierbarer Copolymerisate, die z.B. für wäßrige Sekundärdispersionen geeignet sind. Monomere mit salzbildenden Gruppen sind insbesondere Itaconsäure, Acrylsäure und Methacrylsäure.

Der Gewichtsanteil der weiteren Comonomeren im Copolymerisat kann 0 bis 50 %, vorzugsweise 0 bis 20 % und ganz besonders bevorzugt 0 bis 10 Gew.-% betragen.

Die Herstellung des Copolymerisats erfolgt durch radikalische Polymerisation. Geeignete Polymerisationsmethoden, wie Substanz-, Lösungs-, Suspensions- oder Emulsionspolymerisation sind dem Fachmann bekannt.

Bei der Lösungspolymerisations wird eine Lösung des Copolymerisat in einem organischen Lösungsmittel erhalten.

Vorzugsweise wird das Copolymerisat durch Lösungspolymerisation mit anschließender Dispergierung in Wasser oder besonders bevorzugt durch Emulsionspolymerisation hergestellt, so daß wäßrige Copolymerdispersionen entstehen.

Die Emulsionspolymerisation kann diskontinuierlich, mit oder ohne Verwendung von Saatlatices, unter Vorlage aller oder einzelner Bestandteile des Reaktionsgemisches, oder bevorzugt unter teilweiser Vorlage und Nachdosierung der oder einzelner Bestandteile des Reaktionsgemisches, oder nach dem Dosierverfahren ohne Vorlage durchgeführt werden.

Die Comonomeren können bei der Emulsionspolymerisation wie üblich in Gegenwart eines wasserlöslichen Initiators und eines Emulgators bei vorzugsweise 30 bis 95°C polymerisiert werden.

Geeignete Initiatoren sind z.B. Natrium-, Kalium- und Ammoniumpersulfat, tert.-Butylhydroperoxide, wasserlösliche Azoverbindungen oder auch Redoxinitiatoren wie H₂O₂/Ascorbinsäure.

Als Emulgatoren dienen z.B. Alkalisalze von längerkettigen Fettsäuren, Alkylsulfate, Alkylsulfonate, alkylierte Arylsulfonate oder alkylierte Biphenylethersulfonate. Des weiteren kommen als Emulgatoren Umsetzungsprodukte von Alkylenoxiden, insbesondere Ethylen- oder Propylenoxid mit Fettalkoholen, -säuren oder Phenol, bzw. Alkylphenolen in Betracht.

Im Falle von wäßrigen Sekundärdispersionen wird das Copolymerisat zunächst durch Lösungspolymerisation in einem organischen Lösungsmittel hergestellt und anschließend unter Zugabe von Salzbildnern, z.B. von Ammoniak zu Carbonsäuregruppen enthaltenden Copolymerisaten, in Wasser ohne Verwendung eines Emulgators oder Dispergierhilfsmittels dispergiert. Das organische Lösungsmittel kann abdestilliert werden. Die Herstellung von wäßrigen Sekundärdispersionen ist dem Fachmann bekannt und z.B. in der DE-A-37 20 860 beschrieben.

Zur Einstellung des Molekulargewichts können bei der Polymerisation Regler eingesetzt werden. Geeignet sind z.B. -SH enthaltende Verbindungen wie Mercaptoethanol, Mercaptopropanol, Thiophenol, Thioglycerin, Thioglykolsäureethylester, Thioglykolsäuremethylester und tert.-Dodecylmercaptan.

Die Art und Menge der Comonomeren wird zweckdienlicherweise so gewählt, daß das erhaltene Copolymerisat eine Glasübergangstemperatur zwischen bevorzugt -60 und +140°C, besonders bevorzugt -30 und +80°C und ganz besonders bevorzugt, vor allem im Falle der Verwendung als Klebstoff, zwischen -30 und +20°C hat. Die Glasübergangstemperatur des Copolymerisats läßt sich nach üblichen Methoden wie Differentialthermoanalyse oder Differential Scanning Calorimetrie (s. z.B. ASTM 3418/82, sog. "midpoint temperature") bestimmen.

Im Falle, daß das Copolymerisat nicht selbstvernetzend ist, also keine Monomere c) enthält, kann dem Copolymerisat geeigneterweise für die Vernetzung ein Vernetzungsmittel zugegeben werden. Bei dem Vernetzungsmittel handelt es sich üblicherweise um eine Verbindung, welche mindestens zwei Keto- oder Aldehydgruppen, bzw. mindestens eine Keto- und eine Aldehydgruppe enthält.

Solche Verbindungen sind z.B. Succindialdehyd, Glutardialdehyd oder Terephthaldialdehyd.

Geeignet als Vernetzungsmittel sind insbesondere auch radikalische Copolymerisate - im folgenden auch polymeres Vernetzungsmittel genannt - welche voranstehend genannte Monomere c) einpolymerisiert enthalten.

In Betracht kommen z.B. polymere Vernetzungsmittel, die aus 30 - 99,9 Gew.-%, vorzugsweise 70 - 99,9 Gew.-% der Monomeren b), 0,1 - 30 Gew.-%, vorzugsweise 0,1 - 10 Gew.-% der Monomeren c) und 0 - 50 Gew.-%, vorzugsweise 0 - 20 Gew.-% der Monomeren d) aufgebaut sind. Über die Art der Monomeren, die Glasübergangstemperatur und die Herstellung gilt vorzugsweise das voranstehend über obige Copolymerisate gesagte.

Des weiteren können die erfindungsgemäßen Copolymerisate auch mit Hydroxylgruppen enthaltenden Verbindungen, insbesondere auch Hydroxylgruppen-enthaltenden Copolymerisaten vernetzen.

Das Vernetzungsmittel, soweit gewünscht, wird vorzugsweise zur Lösung oder Dispersion der Copolymerisate gegeben.

Es ist jedoch auch möglich, das Copolymerisat und das Vernetzungsmittel erst bei der Anwendung, z.B. der Beschichtung von Oberflächen zusammenzubringen. Dazu könnte auf die Oberfläche z.B. zunächst das Vernetzungsmittel als sog. Primer aufgebracht werden und anschließend die Beschichtung mit der Dispersion oder Lösung der Copolymerisate erfolgt.

Die Lösung oder Dispersion der erfindungsgemäßen Copolymerisate eignet sich unter anderem für die Verwendung als Anstrich- und Beschichtungsmittel z.B. für Kunststoff-, Holz- oder Metalloberflächen, für Oberflächen mineralischer Baustoffe wie Beton oder Ton, für asphalt- oder bitumenhaltige Straßenbeläge oder für Textilien, Vliesstoffe, Leder oder Papier. Sie eignen sich ebenfalls für Anwendungen in der Bauchemie z.B. als Klebstoffe, Dichtungsmassen, Bindemittel oder ähnliches.

Die Dispersionen der Lösungen können je nach Verwendungszweck noch übliche Hilfs- und Zusatzmittel enthalten. Hierzu gehören beispielsweise Füllstoffe wie Quarzmehl, Quarzsand, hochdisperse Kieselsäure, Schwerspat, Calciumcarbonat, Kreide, Dolomit oder Talkum, die oft zusammen mit geeigneten Netzmitteln wie z.B. Polyphosphaten wie Natriumhexamethaphosphat, Naphthalinsulfonsäure, Ammonium- oder Natriumpolyacrylsäuresalze eingesetzt werden, wobei die Netzmittel im allgemeinen von 0,2 bis 0,6 Gew.-%, bezogen auf den Füllstoff, zugesetzt werden.

Auch Fungizide zur Konservierung können zugesetzt werden. Diese kommen im allgemeinen in Mengen von 0,02 bis 1 Gew.-%, bezogen auf die Dispersionen oder Lösungen zum Einsatz. Geeignete Fungizide sind beispielsweise Phenol- oder Kresol-Derivate oder zinnorganische Verbindungen.

Besonders eignen sich die Dispersionen oder Lösungen auch als Dichtstoff- oder Klebstoffzubereitungen, insbesondere als Kaschierklebstoff zur Herstellung von Verbundfolien und Glanzfolien. Als solche können sie neben obengenannten Zusatzstoffen noch spezielle, in der Klebstofftechnologie übliche Hilfs- und Zusatzmittel enthalten. Hierzu gehören beispielsweise Verdickungsmittel, Weichmacher oder auch klebrigmachende Harze wie z.B. Naturharze oder modifizierte Harze wie Kolophoniumester oder synthetische Harze wie Phthalatharze.

Die Dispersionen oder Lösungen der selbstvernetzenden oder fremdvernetzenden Copolymerisate, welche noch ein Vernetzungsmittel enthalten, sind lagerstabil. Die Vernetzung tritt bereits bei Raumtemperatur mit Verflüchtigung des Lösungsmittels oder Dispergiermittels ein. Durch die Gegenwart von Metallsalzen werden die Vernetzungsfähigkeit und die guten Haftungseigenschaften der Copolymerisate nicht beeinträchtigt.

Die mit diesen Dispersionen oder Lösungen hergestellten Beschichtungen oder Verklebungen weisen eine gute Chemikalien- oder Lösungsmittelbeständigkeit und eine gute innere Festigkeit (Kohäsion) auf.

Bei den im folgenden beschriebenen Versuchen wird entsalztes Wasser eingesetzt und die Prozentangaben beziehen sich auf das Gewicht, wenn nichts anderes angegeben ist.

### Beispiele

Zunächst wurden die Vorstufen gemäß den allgemeinen Formel III und IV synthetisiert, aus denen dann die erfindungsgemäßen Hydroxylaminderivate hergestellt wurden.
1. O-(2-Acroyloxy-propyl)-hydroxylammoniumchlorid, im folgenden Monomer a1 genannt, der Formel 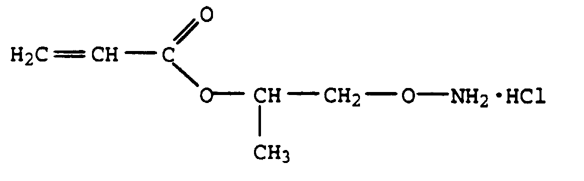
1.1 Vorstufe IV mit R¹ = H, 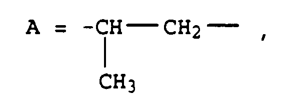 R² = -CH₃ und R³ = -OC₂H₅
In eine Mischung aus 30 ml Diethylether, 32,2 g (0,20 mol) O-(2-Hydroxypropyl)-acethydroxamsäureethylester, 24,2 g (0,24 mol) Triethylamin und 0,10 g 2,6-Ditert.butyl-parakresol (Stabilisator) tropft man bei 10°C (Eiskühlung) eine Lösung aus 120 ml Diethylether und 22,4 g (0,24 mol) Acrylsäurechlorid innerhalb 30 min ein. Man läßt die Reaktionsmischung in 5 h bei Raumtemperatur ausreagieren und schüttelt sie anschließend mit einer Lösung von 33,1 g (0,24 mol) Kaliumcarbonat in 100 g Wasser. Nach dem Abtrennen der Wasserphase wird die Etherlösung im Wasserstrahlvakuum bei Raumtemperatur eingedampft und der Eindampfrückstand im Ölpumpenvakuum destilliert. Man erhält 32,5 g (76 %) O-(2-Acroyloxy-propyl)-acethydroxamsäureethylester, Sdp. 66-68°C/0,1 mbar.
1.2 Monomer a1
In eine Mischung aus 114 ml Dioxan, 45,4 g (0,225 mol HCl) einer 18,1 %igen Lösung von HCl in Dioxan und 4,10 g (0,228 mol) Wasser wurden bei Raumtemperatur innerhalb von 15 min 24,5 g (0,114 mol) des Reaktionsproduktes von 1.1 eingetropft. Man ließ 3 h bei Raumtemperatur reagieren. Dann wurden 570 ml Diethylether eingetropft und nach 20stündiger Kristallisationszeit 19,2 g Produkt, Schmp. 92 bis 94°C (aus Ethanol/Diethylether), durch Abfiltrieren gewonnen. Die Ausbeute betrug 93 % d. Theorie.

| Elementaranalyse | C | H | O | N | Cl |
|---|---|---|---|---|---|
| Berechnet | 39,68 | 6,66 | 26,43 | 7,71 | 19,52 |
| Gefunden | 39,25 | 6,82 | 26,63 | 7,62 | 19,81 |

2. O-(2-Methacroylamidocarbonyloxy-ethyl)-hydroxylammoniumchlorid, im folgenden Monomer a2 genannt, der Formel 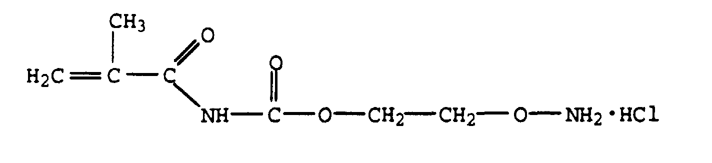
2.1 Vorstufe III mit 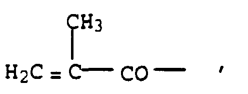 A = -CH₂-CH₂-, R² = -CH₃ und R³ = -OC₂H₅.
In eine Mischung aus 29,4 g (0,20 mol) O-(2-Hydroxyethyl)-acethydroxamsäureethylester, 40 ml Dichlormethan und 0,1 g 2,6-Ditert.butyl-parakresol (Stabilisator) tropft man bei 20 -22°C eine Lösung von 22,2 g (0,20 mol) Methacroylisocyanat in 25 ml Dichlormethan ein und läßt 1 h bei Raumtemperatur nachreagieren. Man dampft das Lösungsmittel bei 30°C im Vakuum ab und kristallisiert den Eindampfrückstand (51,5 g, 100 % Rohausbeute) aus 200 ml Methyl-tert-butylether bei -25°C um. Das reine Produkt wird abfiltriert und schmilzt bei 66-68°C.

| Elementaranalyse | C | H | O | N | |
|---|---|---|---|---|---|
| Formel | 11 | 18 | 5 | 2 | |
| Theorie | 51,16 | 7,03 | 30,97 | 10,85 | Molm.=258,3 |
| Gefunden | 51,1 | 7,0 | 31,0 | 10,6 | |
| Atomverh. | 5,62 | 9,18 | 2,56 | 1,00 | |

2.2 Monomer a2
Eine Mischung aus 30 g Wasser, 15,0 g (0,15 mol) konz. Salzsäure und 19,4 g (0,075 mol) des Reaktionsproduktes von 2.1 wurde 3 h bei Raumtemperatur gerührt. Man dampfte die Lösung bei Raumtemperatur im Vakuum (0,5 mbar) ein und löste den Destillationsrückstand in 20 ml Ethanol auf. Nach Zugabe von 200 ml Diethylether ließ man 20 h bei +5°C kristallisieren. Es wurden 16.2 g (96 % d. Th) Produkt Schmp. 188°C, durch Filtration gewonnen.
3. O-(2-Methacroyloxy-propyl)hydroxylammoniumchlorid, im folgenden Monomer a3 genannt, der Formel 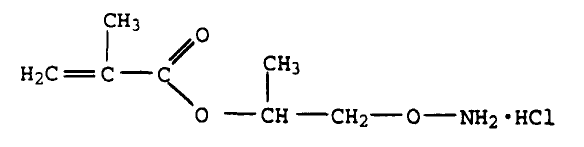
3.1 Vorstufe IV mit R¹ = -CH₃, 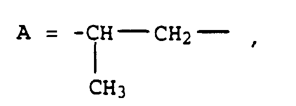 R² = -CH₃ und R³ = -OC₂H₅.
In eine Mischung aus 95 g (0,59 mol) O-(2-Hydroxypropyl)-acethydroxamsäureethylester, 60,2 g (0,59 mol) Triethylamin und 0,1 g Phenothiazin (Stabilisator) wurden bei 80°C 110 g (0,71 mol) Methacrylsäureanhydrid eingetropft. Nach 10stündiger Reaktionszeit bei 80°C wurde die Reaktionsmischung aus 20°C abgekühlt und 45 min mit einer Lösung von 98,5 g (0,71 mol) Kaliumcarbonat in 500 ml Wasser gerührt. Die abgetrennte organische Phase lieferte bei Destillation im Vakuum den O-(2-Methacroyloxy-propyl)-acethydroxamsäureethylester, Sdp. 65-67°C/0,1 mbar.
3.2 Monomer a3
Die Hydrolyse der Vorstufe IV von 3.1 mit Salzsäure erfolgte wie bei der Herstellung des Monomer a1 in Dioxin als Lösungsmittel und lieferte das O-(2-Methacroyloxy-propyl)-hydroxylammonium-chlorid mit dem Schmp. = 75 bis 77°C.

| Elementaranalyse | C | H | N | Cl |
|---|---|---|---|---|
| Berechnet | 42,97 | 7,21 | 7,16 | 18,12 |
| Gefunden | 42,65 | 7,50 | 7,12 | 18,03 |

4. O-(2-Methacroyloxyethyl)-hydroxylammoniumchlorid, im folgenden Monomer a4 genannt, der Formel 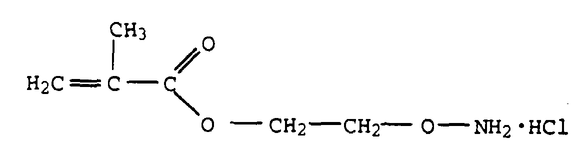
4.1 Vorstufe IV mit R¹ = -CH₃,
A = ―CH₂―CH₂―,
R² = - CH₃ und R³ = -OC₂H₅.

Die Herstellung des O-(2-Methacroyloxy-ethyl)acethydroxamsäureethylesters aus O-(2-Hydroxyethyl)-acethydroxamsäureethylester und Methacrylsäureanhydrid erfolgte entsprechend der Vorstufe von 3.1, jedoch bei einer Reaktionstemperatur von 64°C. Sdp. 65°C/0,1 mbar.

| Elementaranalyse | C | H | O | N | |
|---|---|---|---|---|---|
| Formel | 10 | 17 | 4 | 1 | |
| Theorie | 55,80 | 7,96 | 29,73 | 6,51 | Molm.=215,2 |
| Gefunden | 55,8 | 8,1 | 29,4 | 6,9 | |
| Atomverh. | 9,43 | 16,31 | 3,73 | 1,00 | |

4.2 Monomer a4
Die Hydrolyse der Vorstufe von 4.1 wurde entsprechend der Arbeitsvorschrift von 2.2 (Monomer a2) durchgeführt und lieferte das O-(2-Methacroyloxy-ethyl)-hydroxylammoniumchlorid vom Schmp. 84-86°C.

### Herstellung der Dispersionen

Die Polymerisationen wurden in üblichen Rührkesseln unter Rühren durchgeführt.

### Dispersion Typ 1

Man legte ein Gemisch aus 250 g Wasser, 40 g einer 15 gew.-%igen wäßrigen Lösung von Natriumlaurylsulfat, 15 g einer 40 gew.-%igen wäßrigen Lösung von mit 50 Mol Ethylenoxid ethoxiliertem Nonylphenol, 21,3 g von Zulauf 1 und 10,1 g von Zulauf 2 vor und erwärmte unter Rühren auf 75°C. Nach dem Anpolymerisieren führte man den restlichen Zulauf 1 mit konstanter Geschwindigkeit zu dem Polymerisationsgemisch.

Nach Beendigung von Zulauf 1 wurden 6 g einer 25 gew.-%igen wäßrigen Ammoniaklösung zugegeben. Danach wurde Zulauf 3 in 55 Minuten mit konstanter Geschwindigkeit zugefahren. Zulauf 2 wurde beginnend mit dem Start des restlichen Zulaufs 1 innerhalb von 2,5 Stunden mit gleichbleibender Geschwindigkeit zugegeben. Danach wurde noch eine Stunde bei gleichbleibender Temperatur nachpolymerisiert und anschließend abgekühlt und filtriert.

### Zulauf 1 (gerührt):

- 108,3 g: Wasser
- 7,5 g: 40 gew.-%ige Lösung von mit 50 Mol Ethylenoxidethoxiliertem Nonylphenol,
- 300 g: n-Butylacrylat
- 30 g: Methylmethacrylat
- 9 g: Acrylsäure
- 3 g: 50 gew.-%ige wäßrige Lösung von Acrylamid

### Zulauf 2:

- 100 g: Wasser
- 0,6 g: Natriumperoxodisulfat

### Zulauf 3 (gerührt):

- 74,3 g: Wasser
- 40 g: 15 gew.-%ige wäßrige Lösung von Natriumlaurylsulfat,
- 7,5 g: 40 gew.-%ige wäßrige Lösung von mit 50 Mol Ethylenoxid ethoxiliertem Nonylphenol
- 24 g: n-Butylacrylat
- 222 g: Methylmethacrylat
- 12 g: Monomer a)
- 3 g: 50 gew.-%ige wäßrige Lösung von Acrylamid.

In dieser Weise wurden die Dispersionen D1 bis D4 (s. Tabelle) hergestellt.

### Dispersion Typ 2

### Man verfährt wie bei Dispersion Typ 1.

### Zulauf 1: wie bei D1

### Zulauf 2: wie bei D1

### Zulauf 3 (gerührt):

- 50,3 g: Wasser
- 40 g: 15 gew.-%ige Lösung von Natriumlaurylsulfat
- 7,5 g: 40 gew.-%ige wäßrige Lösung von mit 50 Mol Ethylenoxid ethoxiliertem Nonylphenol
- 24 g: n-Butylacrylat
- 222 g: Methylmethacrylat
- 6 g: Monomer a)
- 30 g: 20 gew.-%ige wäßrige Lösung Diacetonacrylamid
- 3 g: 50 gew.-%ige wäßrige Lösung von Acrylamid.

In dieser Weise wurden die Dispersionen D5 bis D8 hergestellt.

### Hilfsdispersion HD

200 g Wasser, 37 g des Zulaufs 1 und 20 g des Zulaufs 2 wurden vorgelegt und auf 85°C erwärmt. Nach 15 Minuten wurde innerhalb von 2 h gleichmäßig der Zulauf 1 sowie innerhalb von 2,5 h gleichmäßig der Zulauf 2 zum Reaktionsgefäß zugegeben. Nach der letzten Initiator-Zugabe (Zulauf 2) wurde die Dispersion noch 1 Stunde bei 85°C gerührt.

### Zulauf 1: (dieser Zulauf wird während der Polymerisation gerührt)

- 107,5 g: Wasser
- 400 g: Ethylacrylat
- 90 g: Methylmethacrylat
- 50 g: 20 gew.-%ige wäßrige Diacetonacrylamid-Lösung
- 50 g: 20 gew.-%ige Lösung von Natriumlaurylsulfat
- 50 g: 20 gew.-%ige Lösung des Umsetzungsprodukts von p-Isononylphenol mit ca. 50 Mol Ethylenoxid in Wasser

### Zulauf 2:

- 100 g: Wasser
- 3 g: Natriumpersulfat

### Anwendungstechnische Prüfungen

### Vernetzbarkeit (Prüfung durch Quellungsverhalten und Bestimmung der extrahierbaren Anteile)

Je 10 g der Dispersionen bzw. Dispersionsmischungen gemäß der Tabelle wurden verfilmt und die Filme 1 Woche bei Raumtemperatur getrocknet. Anschließend wurde das Quellungsverhalten als Maß für den Vernetzungsgrad dieser Filme in Tetrahydrofuran untersucht, indem ca. 1 g der verfilmten Proben in Tetrahydrofuran 24 h gelagert und die Lösungsmittelaufnahme in % gemessen wurde.

Bei vernetzten Polymeren kommt es durch Aufnahme von Lösungsmittel zur Quellung. Mit steigendem Vernetzungsgrad wird die Quellung geringer, da weniger Lösungsmittel vom eng vernetzten Polymer aufgenommen werden kann. Nicht oder kaum vernetzte Polymere werden durch Lösungsmittel zum großen Teil gelöst oder quellen bei Vorhandensein einer geringen Anzahl von Vernetzungsstellen übermäßig stark an.

Die extrahierbaren Anteile wurden bestimmt durch Rückwiegen bei Raumtemperatur nach Trocknung im Trockenschrank bei 80°C während 4 h.

Die Ergebnisse sind in der Tabelle angegeben.

### Verklebungseigenschaften bei der Glanzfolienkaschierung

Die Dispersionen bzw. Dispersionsmischungen der Tabelle wurden mit einer Trockenschichtdicke von 5 g/m² auf mit Offsetfarben bedruckten Kartonagen gerakelt und nach ca. 30 sec mit biaxial verstreckten Polypropylenfolien oder Acetatfolien kaschiert.

Nach 6 Wochen Lagerung bei Raumtemperatur wurde geprüft, ob es beim Abreißen der Folie zu einem Farbausriß von der Kartonage kommt und ob sich im Bereich von Nuten (Prägungen in der Kartonage) die Folie ablöst bzw. nicht fest anliegt (Nutstandfestigkeit).

Alle Zusammensetzungen und Ergebnisse sind in der folgenden Tabelle zusammengefaßt.

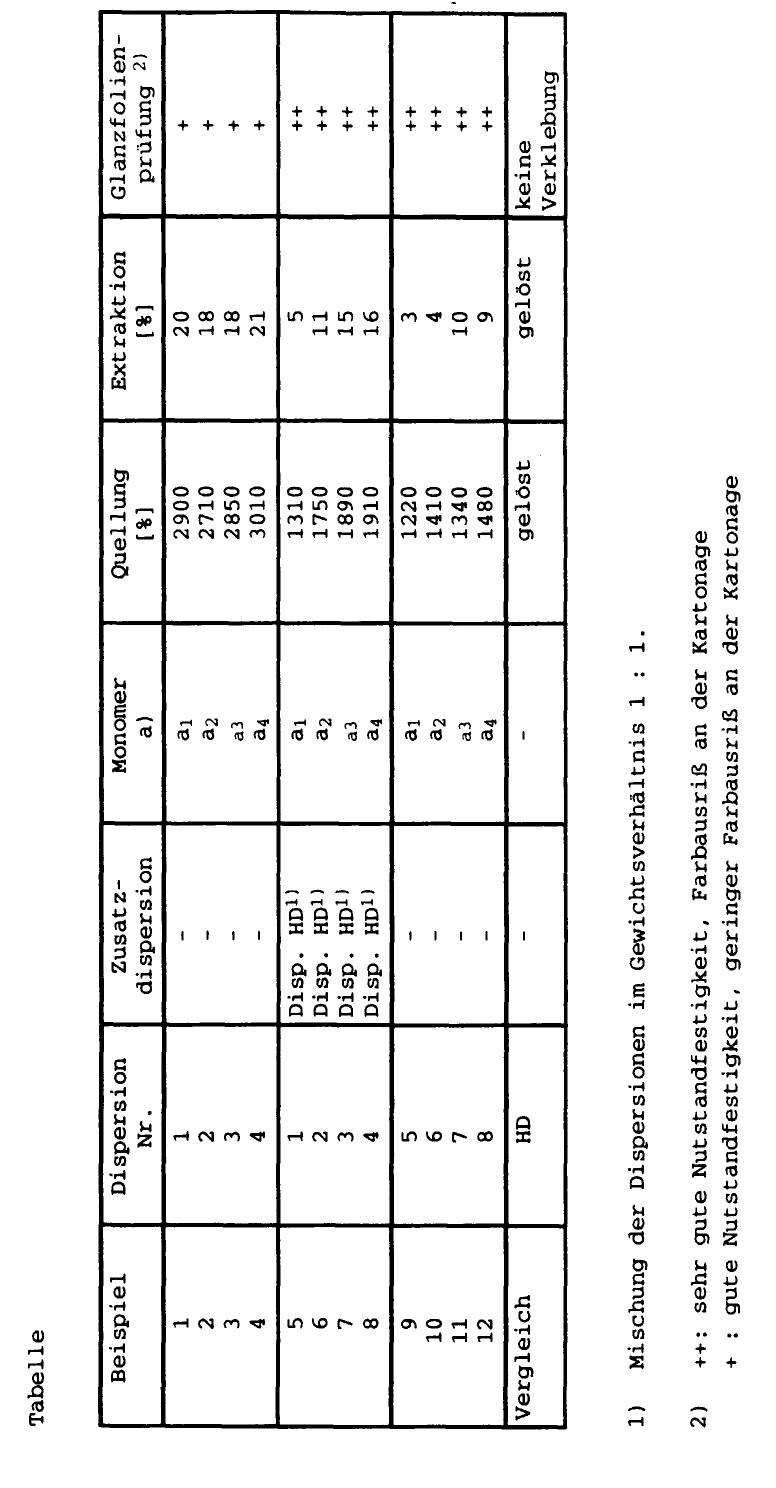

## Patentansprüche

1. Hydroxylaminderivate der allgemeinen Formel 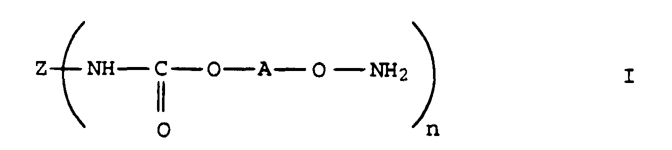 oder 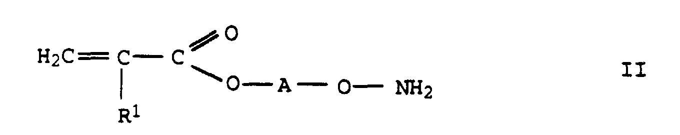 worin A für ein zweiwertiges Bindeglied steht, R¹ ein Wasserstoffatom oder eine C₁-C₄-Alkylgruppe sein kann, Z für einen n-wertigen organischen Rest steht, der eine copolymerisierbare ethylenisch ungesättigte Gruppe enthält und n eine ganze Zahl von 1 bis 3 ist, und deren Salze.

2. Verfahren zur Herstellung von Hydroxylaminderivaten und deren Salzen gemäß Anspruch 1, dadurch gekennzeichnet, daß man copolymerisierbare Oximether der Formeln 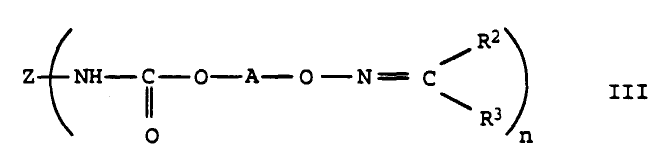 oder 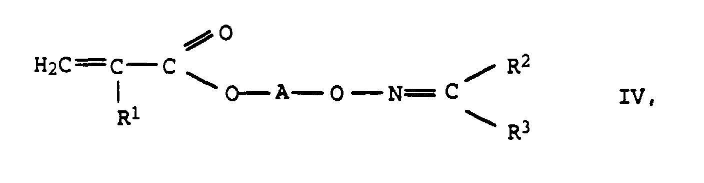 worin
R² und R³ unabhängig voneinander für einen C₁- bis C₁₀-Alkyl, einen C₁-C₁₀-Alkoxy-, einen C₅- bis C₁₀-Cycloalkyl- oder einen C₅- bis C₁₀-Arylrest, welche auch 1 - 3 nicht benachbarte Stickstoff-, Sauerstoff- oder Schwefelatome als Heteroatome in der Kohlenstoffkette oder im Kohlenstoffring enthalten können und durch ein bis drei C₁- bis C₄-Alkyl- oder -Alkoxygruppen substituiert sein können, stehen, R² oder R³ für ein Wasserstoffatom stehen können oder R² oder R³ gemeinsam eine Brücke aus 3 bis 14 Kohlenstoffatomen bilden, wobei ein Teil der Kohlenstoffatome auch Bestandteil eines aromatischen Rings sein kann,
mit Wasser und einer Säure mit einer Säurekonstanten >10⁻² umsetzt und gegebenenfalls deprotoniert.

3. Verfahren nach Anspruch 2, dadurch gekennzeichnet, daß man die Umsetzung bei 0 bis 50°C durchführt.

4. Copolymerisate, enthaltend als Aufbaukomponente 0,01 bis 30 Gew.-% der Hydroxylaminderivate in freier und/oder in Salzform gemäß Anspruch 1.

5. Copolymerisate nach Anspruch 4, enthaltend als Aufbaukomponenten
a) 0,01 bis 30 Gew.-% eines Hydroxylaminderivats der Formel I oder II in freier Form und/oder in Salzform
b) 30 bis 99,99 Gew.-% mindestens eines C₁- bis C₂₀-Alkyl(meth-)acrylats, eines Vinylesters von 1 bis 20 C-Atome enthaltenden Carbonsäuren, eines Vinylaromaten mit bis zu 20 C-Atomen, eines ethylenisch ungesättigten Nitrils mit 3 bis 6 C-Atomen, eines Vinylhalogenids oder eines nichtaromatischen Kohlenwasserstoffs mit 4 bis 8 C-Atomen und mindestens 2 konjungierten Doppelbindungen,
c) 0 bis 30 Gew.-% mindestens eines Comonomeren mit mindestens einer Keto- oder Aldehydgruppe und
d) 0 bis 50 Gew.-% mindestens eines weiteren Monomeren.

6. Verwendung von Copolymerisaten gemäß Anspruch 4 oder 5 als Beschichtungsmittel.

7. Verwendung von Copolymerisaten gemäß Anspruch 4 oder 5 als Klebstoffe.

8. Beschichtete Substrate, erhältlich unter Verwendung von Copolymerisaten gemäß Anspruch 4 oder 5.

9. Verklebter Verbund, erhältlich unter Verwendung von Copolymerisaten gemäß Anspruch 4 oder 5.

## Claims

1. A hydroxylamine derivative of the general formula 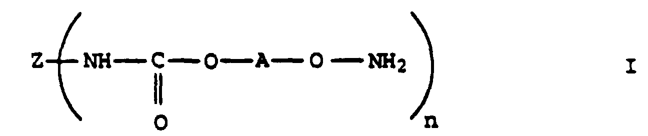 or 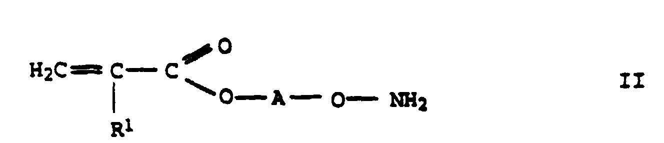 in which A is a divalent linking member, R¹ can be a hydrogen atom or a C₁-C₄-alkyl group, Z is an n-valent organic radical containing a copolymerizable ethylenically unsaturated group, and n is an integer from 1 to 3, or a salt thereof.

2. A process for preparing a hydroxylamine derivative or salt thereof as claimed in claim 1, which comprises reacting a copolymerizable oxime ether of the formulae 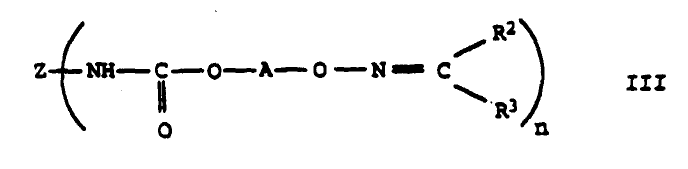 or 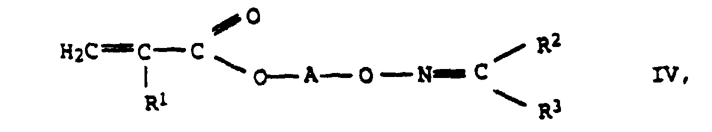 in which R² and R³ independently of one another are a C₁- to C₁₀-alkyl, a C₁-C₁₀-alkoxy radical, a C₅- to C₁₀-cycloalkyl radical or a C₅- to C₁₀-aryl radical, which may also contain 1 - 3 nonadjacent nitrogen atoms, oxygen atoms or sulfur atoms as heteroatoms in the carbon chain or in the carbon ring and can be substituted by from one to three C₁- to C₄-alkyl or -alkoxy groups, R² or R³ can be a hydrogen atom, or R² or R³ together form a bridge comprising 3 to 14 carbon atoms, it also being possible for some of the carbon atoms to be part of an aromatic ring, with water and an acid having an acid constant >10⁻² and carrying out deprotonation if desired.

3. A process as claimed in claim 2, wherein the reaction is carried out at from 0 to 50°C.

4. A copolymer containing as structural component from 0.01 to 30% by weight of a hydroxylamine derivative in free form and/or in salt form as claimed in claim 1.

5. A copolymer as claimed in claim 4, containing as structural components
a) from 0.01 to 30% by weight of a hydroxylamine derivative of the formula I or II in free form and/or in salt form
b) from 30 to 99.99% by weight of at least one C₁- to C₂₀-alkyl (meth) acrylate, a vinyl ester of carboxylic acids containing 1 to 20 carbon atoms, a vinyl aromatic compound having up to 20 carbon atoms, an ethylenically unsaturated nitrile having 3 to 6 carbon atoms, a vinyl halide or a nonaromatic hydrocarbon having 4 to 8 carbon atoms and at least 2 conjugated double bonds,
c) from 0 to 30% by weight of at least one comonomer containing at least one keto group or aldehyde group, and
d) from 0 to 50% by weight of at least one further monomer.

6. The use of a copolymer as claimed in claim 4 or 5 as a coating agent.

7. The use of a copolymer as claimed in claim 4 or 5 as an adhesive.

8. A coated substrate obtainable using a copolymer as claimed in claim 4 or 5.

9. An adhesively bonded composite structure obtainable using a copolymer as claimed in claim 4 or 5.

## Revendications

1. Dérivés de l'hydroxylamine de la formule générale : 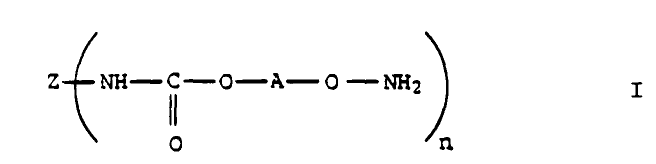 ou 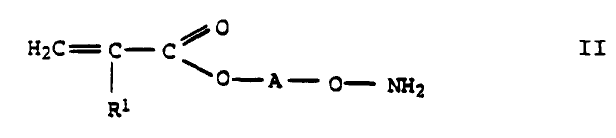 dans laquelle A représente un maillon de liaison bivalent, R¹ représente un atome d'hydrogène ou un radical alkyle en C₁-C₄, Z représente un reste organique n-valant, qui contient un groupe éthyléniquement insaturé copolymérisable et n représente un nombre entier dont la valeur varie de 1 à 3 et leurs sels.

2. Procédé de préparation de dérivés de l'hydroxylamine et de leurs sels suivant la revendication 1, caractérisé en ce que l'on fait réagir et déprotone éventuellement des oximéthers copolymérisables des formules : 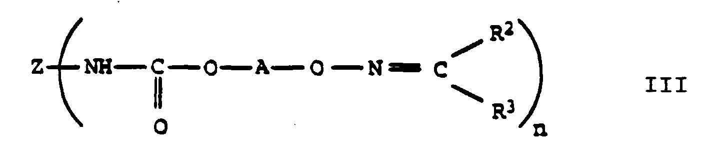 ou 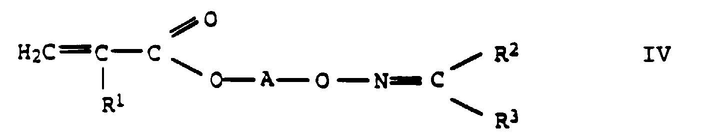 dans lesquelles
R² et R³ représentent, chacun indépendamment l'un de l'autre, un radical alkyle en C₁-C₁₀, alcoxy en C₁-C₁₀, cycloalkyle en C₅-C₁₀, aryle en C₅-C₁₀, qui peuvent également contenir de 1 à 3 atomes d'azote, d'oxygène ou de soufre, non voisins, à titre d'hétéroatomes, dans la chaîne hydrocarbonée ou dans le noyau hydrocarboné et qui peuvent être substitués par de un à trois radicaux alkyle en C₁-C₄ ou alcoxy en C₁-C₄, R² ou R³ représente chacun un atome d'hydrogène, ou bien R² et R³ forment en commun un pont constitué de 3 à 14 atomes de carbone, où une partie des atomes de carbone peuvent aussi être parties constituantes d'un noyau aromatique,
avec de l'eau et un acide possédant une constante d'acidité supérieure à 10⁻².

3. Procédé suivant la revendication 2, caractérisé en ce que l'on entreprend la réaction à une température de 0 à 50°C.

4. Copolymères, qui contiennent, à titre de composant constitutif, 0,01 à 30% en poids des dérivés de l'hydroxylamine sous forme libre et/ou sous forme de sel, suivant la revendication 1.

5. Copolymères suivant la revendication 4, qui contiennent, à titre de composants constitutifs,
a) 0,01 à 30% en poids d'un dérivé de l'hydroxylamine de la formule I ou de la formule II, sous forme libre et/ou sous forme de sel;
b) 30 à 99,99% en poids d'au moins un (méth)acrylate d'alkyle en C₁-C₂₀, d'un ester vinylique d'acides carboxyliques contenant de 1 à 20 atomes de carbone, d'un composé vinyle aromatique comportant jusqu'à 20 atomes de carbone, d'un nitrile éthyléniquement insaturé comportant de 3 à 6 atomes de carbone, d'un halogénure de vinyle ou d'un hydrocarbure non aromatique comportant de 4 à 8 atomes de carbone et au moins deux doubles liaisons conjuguées,
c) 0 à 30% en poids d'au moins un comonomère avec au moins un groupe céto ou aldéhyde, et
d) 0 à 50% en poids d'au moins un autre monomère.

6. Utilisation des copolymères suivant la revendication 4 ou 5, à titre d'agents de revêtement.

7. Utilisation des copolymères suivant la revendication 4 ou 5, à titre de colles.

8. Supports revêtus, que l'on peut obtenir par l'utilisation de copolymères suivant la revendication 4 ou 5.

9. Lamifié ou stratifié collé, que l'on peut obtenir par l'utilisation de copolymères suivant la revendication 4 ou 5.
